# EUROPEAN PATENT APPLICATION

(11) **EP 4 230 230 A1**
(43) Date of publication of application: **23.08.2023**
(21) Application number: 21879864.3
(22) Date of filing: 27.09.2021
(51) Int. Cl.: A61L 9/16, F24F 7/003

(54) **AIR PURIFICATION DEVICE, AIR PURIFICATION METHOD, AND AIR PURIFICATION SYSTEM**

(30) Priority: 16.10.2020 JP 2020174713
(71) Applicant: Takahashi, Fumio, Hitachi-shi, Ibaraki 319-1224 (JP)
(72) Inventor: Takahashi, Fumio, Hitachi-shi, Ibaraki 319-1224 (JP)
(74) Representative: Cross, James Peter Archibald
(86) International application number: PCT/JP2021/035298
(87) International publication number: WO 2022/080123

(57) **Abstract**

An air cleaner comprises a housing body 6 provided with an inlet opening for sucking an external air, a filter 2 received in the housing body 6 for purifying air, a blowing member 3 for blowing out the air, a drive portion 4 for rotation-driving the blowing member 3, and a diffusion member 7 provided to the housing body 6 as outlet opening for blowing out the purified air purified through the filter 2 to the exterior, wherein the inlet opening and the outlet opening are adjacent to each other, and the air purified through the filter 2 is diffused and blown out by the diffusion member 7, thereby an air cleaner suitable for preventing virus infection from person to person.

## Description

### Technical Field

The present invention relates to an air cleaner, an air cleaning method and an air cleaning system.

### Background Art

An air cleaner is a device for removing dust, pollen, virus and the like floating in an air, from the air and supplying a purified air, and there are known air cleaners of a type by which a drawn-in air is filtered and a purified air is blown out, or a type in which ions are added to the purified air in order to enhance a cleaning efficiency.

Generally, it is said that a technical problem of the air cleaner resides in that a wide range of air space is purified by one air cleaner. Patent Document 1 demonstrates a configuration suitable for drawing a wide range of polluted air. Patent Document 2 demonstrates a configuration for blowing cleaned air over a wide area. Non-patent Document 1 shows a concept that an air is circulated in an entire room by blowing out and drawing in the air to remove dust floating in the far distance.

### PRIOR ART REFERENCE

### Patent Document

Patent Document 1: Japan Patent No. 6724220.
Patent Document 2: Japan Application Laid-open No. 2017-133829

Non-Patent Document 1: SHARP AIR CLEANER pamphlet
(KC-J50/FU-J50/FU-J30)

### SUMMARY OF THE INVENTION

### THE OBJECT TO BE SOLVED BY THE INVENTION

In 2020, the spread of the new coronavirus became a global social problem. Infectious disease epidemics have occurred many times, represented by the plague 100 years ago. In the present-day when the society has become globalized and transportation of people and a thing has been so developed, risk of prevalence of infection diseases has increased year to year. It is said that COVIT-19 virus is transmitted by airborne droplets generated by sneezing and the like. Fine droplets are moved by a flow of air, so holding the air in purified state is effective to prevent infection. This is a reason why an air cleaner for removing effectively droplets as well as viruses floating in the air.

The conventional air cleaner as described above, is generally suitable for cleaning a wide area in a room, and the air purified by the air cleaner is blown out vigorously and reaches far away. The air blown out of the air cleaner, while entraining the air in the room, is returned to the air cleaner, thereby airborne droplets, viruses and the like being removed so that a wide area in the room is purified.

However, with conventional air cleaners for purifying a wide area in the room, when multiple people gather in a room, there is a risk that droplets and viruses generated by an infected person may reach people downwind of the infected person and infect them.

In view of the above problem being taken into consideration, an object of the present invention is to provide an air cleaner, an air cleaning method and an air cleaning system suitable for preventing virus infection from person to person.

### MEANS FOR SOLVING THE PROBLEM

In order to solve the problem, the present invention provides an air cleaner which comprises:
a housing body provided with an inlet opening for sucking an external air;
a filter received in the housing body to purify the air;
a blowing device received in the housing body to blow out the air;
a drive portion for rotation-driving said blowing device;
a diffusion member provided to the housing body as outlet opening for blowing the air purified through said filter to outside, wherein
said inlet opening and said outlet opening are adjacent to each other; and
the air purified through said filter is diffused by said diffusion member and blown out.

Further, the present invention provides an air cleaning method characterized in arranging said air cleaner between person and person.

Further, the present invention provides an air cleaning system characterized in distributed arrangement of a plurality of said air cleaners.

Further, the present invention provides an air cleaning system characterized in arranging a plurality of said air cleaners each for purifying surrounding air locally.

### Advantageous Effects of the Invention

According to the present invention, there can be provided an air cleaner, an air cleaning method and an air cleaning system suitable for preventing virus infection from person to person.

### Brief Description of the Drawings

[Fig. 1] Fig. 1 is a sectional view showing a constitution of an air cleaner relating to a first embodiment of the present invention.
[Fig. 2] Fig. 2 shows a constitution of a diffusion member of the air cleaner relating to the first embodiment of the present invention. Fig. 2A is an enlarged cross-sectional view of a hole in the diffusion member, and Fig. 2B is a partially enlarged view of an inner-side surface of the diffusing member.
[Fig. 3] Fig. 3A is a sectional view showing a constitution of an air cleaner relating to a second embodiment of the present invention, and Fig. 3B is a partially enlarged view of Fig. 3A.
[Fig. 4] Fig. 4A is a view of an inside of the housing body of the air cleaner relating to the second embodiment, when viewed from above, and Fig. 4B is a view showing modified support plates supporting diffusion guides in the second embodiment.
[Fig. 5] Fig. 5 is a sectional view showing a modification of a housing body in the second embodiment.
[Fig. 6] Fig.6A is a view showing an air cleaning system relating to a third embodiment of the present invention, and Fig. 6B is a view showing a modification of the air cleaning system relating to the third embodiment.
[Fig.7] Fig.7A is a sectional view showing a constitution of an air cleaner relating to a fourth embodiment of the present invention, and Fig. 7B is a partially enlarged view of Fig. 7A.
[Fig. 8] Fig. 8 is a view of an inside of a diffusing member of the air cleaner relating to the fourth embodiment, when viewed from above.
[Fig.9] Fig.9A is a sectional view showing a constitution of an air cleaner relating to a fifth embodiment of the present invention, and Fig.9B is a view of an inside of a diffusing member of the air cleaner relating to the fifth embodiment, when viewed from above.
[Fig.10] Fig.10A is a sectional view showing a constitution of an air cleaner relating to a sixth embodiment of the present invention, and Fig.10B is a view of an inside of a housing body of the air cleaner relating to the sixth embodiment, when viewed from above.

### Embodiments for Carrying out the Invention

The object of the present invention is achieved by an air cleaner having a function for purifying mainly an air surrounding closely the air cleaner. This function can be obtained by blowing the diffused air over a wide angle out of the outlet opening of the air cleaner and arranging the inlet opening of the air close to the outlet opening.

Concept of design requirements of the air cleaner having such a function will be explained in detail. The air cleaner is an apparatus which sucks an air, purify the sucked air through a filter, and thereafter blows out the purified air. Since the amount of impurities removed by the filter is small, substantially the same amount of air is blowed out from the air cleaner as is inhaled. The outside air flow of the air cleaner can be simplified and modelled as potential flow from one point of the blowing points to one point of the sucking points. In this potential flow, if the radius (the distance from the center of gravity of the two points) is sufficiently large compared to the distance between the blowing point and the sinking point, the velocity at the radial position is inversely proportional to the cube of the radius. When the radius becomes smaller and less than the distance between the blowing point and the sucking point, the near field becomes a near-field with a large velocity affected by the positions of the blowing point and the sucking point. Assuming a finite room, the first design requirement is to reduce the area of the near-field by bringing the inlet and the outlet as close as possible.

The second design requirement is to prevent jets from being generated at the blowout portion. When air blows out into a wide space, it often becomes a jet that goes straight in a specific direction and reaches farther than the previous potential flow. In order to prevent the occurrence of jets, the air is blown out by diffusing the flow over a wide angle.

The air cleaner relating to each embodiment of the present invention will be explained with reference to the accompanying drawings.

### (First embodiment)

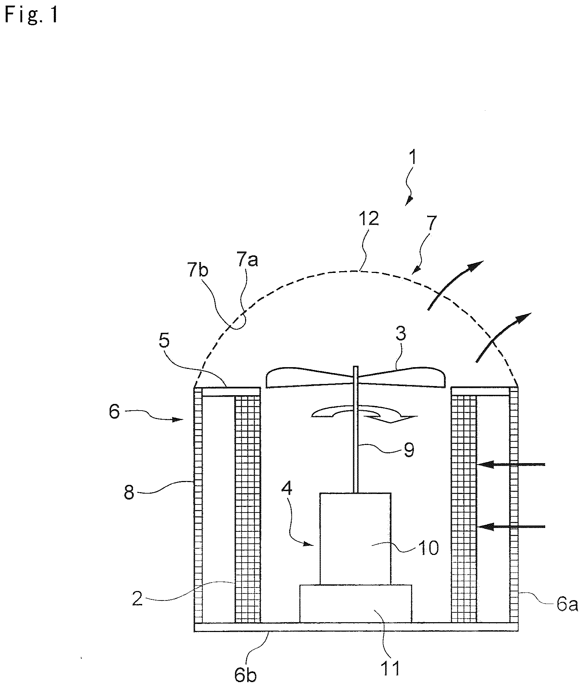

Fig. 1 is a sectional view showing a constitution of the air cleaner relating to the first embodiment of the present invention.

The air cleaner 1 shown in Fig. 1 comprises a filter 2, a fan 3, a drive portion 4, a partition member 5, a housing body 6 receiving them and a diffusion member 7.

The housing body 6 is cylindrical in configuration and closed at an end, and comprises a side wall 6a and a bottom wall 6b. The side wall 6a is formed with a number of micro holes 8 penetrated through the side wall 6a over the entire surface of the side wall 6a, so that it is possible to draw thereinto the air from outside the housing body 6.

The filter 2 is cylindrical in configuration and has a smaller diameter than that of the housing body 6, and is arranged concentrically with the side wall 6a within the housing body 6. The filter 2 is made from porous material of bundles of glass fibers, and purifies the air, that is, removes dust, pollen, virus in the air. Meanwhile, the kind and constitution of the filter 2 are not limited to the one described above.

The fan 3 is arranged in the neighborhood of the upper end of the filter 2 to blow out the air in the housing body 6 upwardly.

The drive portion 4 comprises a motor 10 for driving the fan 3 through a rotary shaft 9, and a power source 11 for supplying electric power to the motor 10. The drive portion 4 is disposed inside the filter 2 within the housing body 6, and the rotary shaft 9 of the fan 3, the filter 2 and the side wall 6a are concentric. Meanwhile, the power source 11 may be a plug-in type or a battery-type.

The partition member 5 comprises an annular plate that horizontally extends from the upper end of the filter 2 to the side wall 6a of the housing body 6 and closes a space formed between the filter 2 and the side wall 6a from upper side. The partition member 5 prevents the air passed through the filter 2 and the air not-passed through the filter 2 from mixing in the housing body 6.

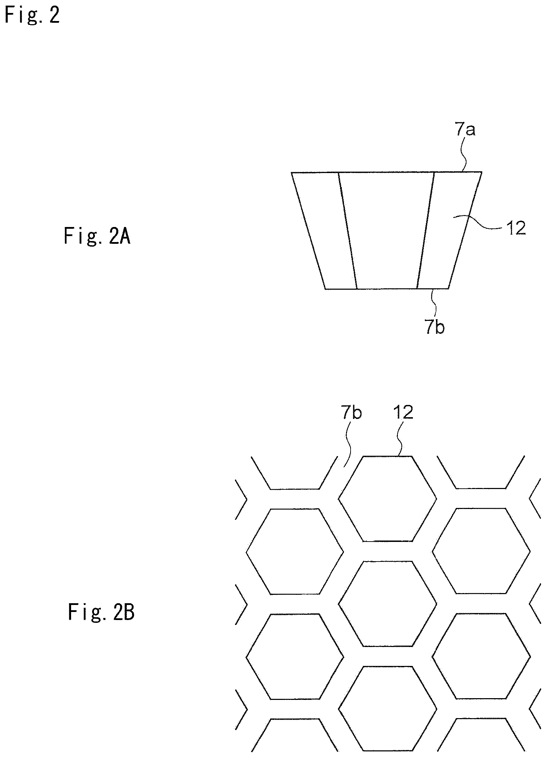

The diffusion member 7 is hemi-spherically hollowed and made from porous material. The diffusion member 7 is formed with a number of holes 12 over the entire surface of the diffusion member 7. The holes 12 each is hexagonal through-hole convergingly extended from the outer side surface 7a to the inner side surface 7b of the diffusion member 7, as shown in Fig. 2(a) to Fig. 2(b). The holes 12 are honeycomb arranged. The diffusion member 7 having such a structure can blow out the air in the housing body 6 blown out by the fan 3, into directions perpendicular to the curved surface of the diffusion member 7 with uniformed flow fluxes. Meanwhile, in order to enhance this effect, a porous resistor member may be provided onto the inner side surface 7b of the diffusion member 7. Such constitution of the diffusion member 7 is mere an example and is not limited thereto.

Under such a constitution, in the air cleaner 1 relating to the present embodiment, the fan 3 is rotated by the drive portion 4, thereby the air around the air cleaner 1 being drawn in from through the side wall 6a of the housing body 6. This air is passed through the filter 2 to remove dust, pollen, virus and the like, and is purified. The purified air is blown out upwardly through the fan 3 and diffused through the diffusion member 7 and blown out of the air cleaner 1. Thus, the air cleaner 1 relating to the present embodiment can purify the surrounding air.

Here, in the air cleaner 1 relating to the present embodiment, an inlet opening of the air is formed by the side wall 6a of the housing body 6, and an outlet opening for blowing out the purified air is formed by the diffusion member 7. The inlet opening and the outlet opening are adjacent to each other. In other words, the first design requirement that in the air cleaner 1 the inlet opening and the outlet opening are arranged as close as possible, is satisfied.

Further, in the air cleaner 1 relating to the present embodiment, the purified air is blown out by the diffusion member 7 in the direction perpendicular to the curved surface of the diffusion member 7 with uniformed flow flux. The second requirement that generation of the jet flow at the blowing-out portion advancing straight into a particular direction is prevented, is satisfied.

By satisfying the first and the second design requirements, in the air cleaner 1 relating to the present embodiment, air flow circulating from the outlet opening to the inlet opening is generated around the air cleaner 1 and thereby the air neighboring the air cleaner 1 can be purified. Thus, in the air cleaner 1 relating to the present embodiment, there is no case where droplets or virus generated by an infected person reach a person located downwind and infect the person, as caused by the conventional air cleaner, and virus infection from person to person can be effectively prevented. Concretely, in a case where one air cleaner 1 is disposed between two persons, for example, air located between the two persons, can be purified locally, in other words, the two persons are separated through the purified air sandwiching the air cleaner 1 so that virus infection can be effectively prevented.

### (Second embodiment)

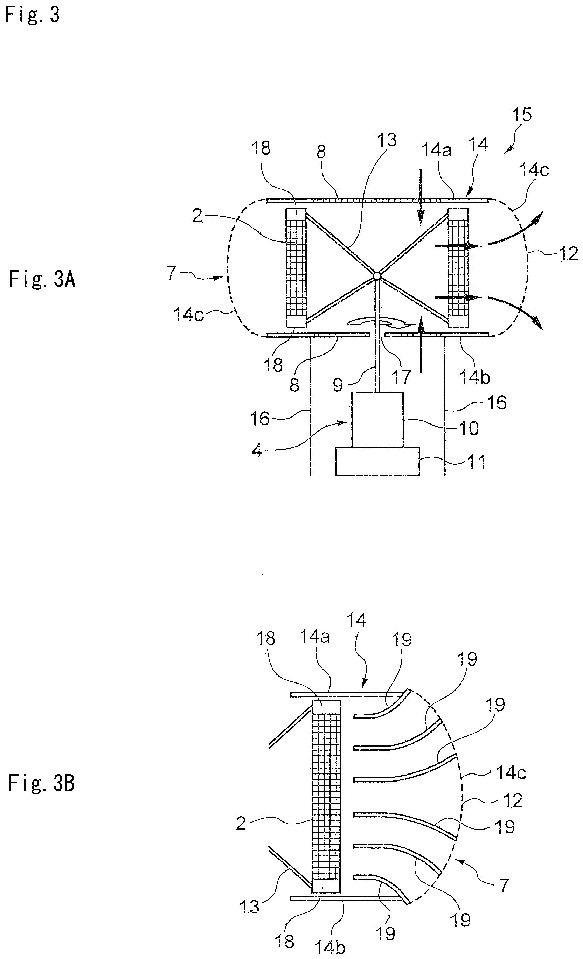

Fig. 3A is a sectional view showing a constitution of an air cleaner relating to the second embodiment of the present invention.

Regarding the air cleaner 15 shown in Fig. 3A, with respect to the same or similar constitution as that of the first embodiment, the same reference signs are put and explanations thereof are omitted, and portions of differing constitution will be explained.

As shown in Fig. 3A, the air cleaner 15 relating to the present embodiment comprises a filter 2, support bars 13 of the filter 2, a housing body 14 for receiving the filter 2 and the support bars 13, a rotary shaft 9, a drive portion 4 and leg 16 for supporting the housing body 14.

The housing body 14 is hollowed, flat and spherically shaped, and more in detail, a section along the center axis is round-cornered rectangle having two sides of equal lengths (upper side and lower side) and two hemispheres.

At a central portion between an upper wall 14a and a lower wall 14b of the housing body 14 (upper face and lower face of the flat sphere), a number of minute pores 8 are formed through the upper wall 14a and the lower wall 14b, so the air outside the housing body 14 can be sucked thereinto. Meanwhile, a round aperture 17 for passing the rotary shaft 9 therethrough is provided at the central portion of the lower wall 14b.

The hemispherical portion 14c of the housing body 14 (a side face of the flat sphere) has a similar constitution to the diffusion member 7 of the first embodiment, and a number of hexagonal holes 12 are honeycomb formed over the entire surface of the hemispherical portion 14c.

In the housing body 14, the cylindrical filter 2 is received rotatably.

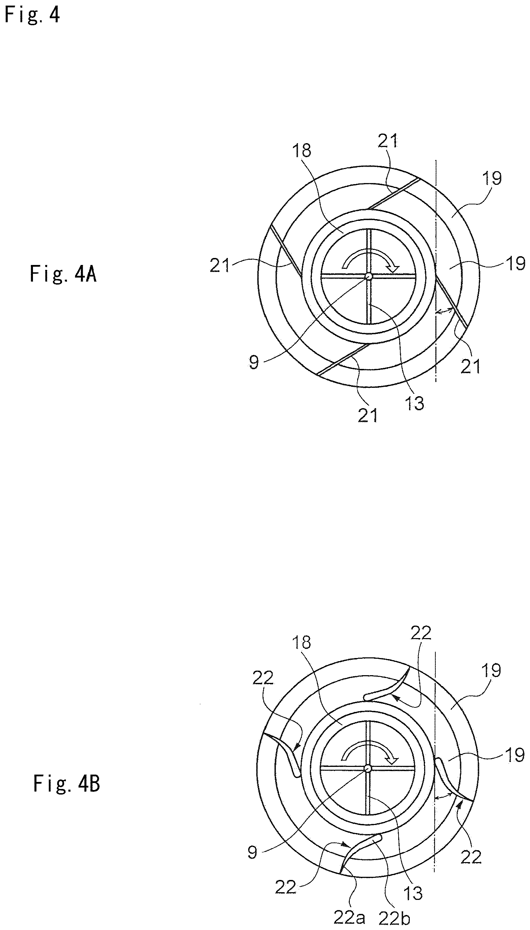

The upper and lower ends of the filter 2 are attached with the ring-shaped plate members 18 and 18 each having the same diameter as that of the filter 2. As shown in Fig. 3A and Fig. 4A, the plate member 18 on the upper end of the filter 2 is fixed to the rotary shaft 8 through four support bars 13, and the plate member 18 on the lower end of the filter 2 is fixed to the rotary shaft 8 in the same way as this. According to this constitution, rotation of the rotary shaft 9 by the drive portion 4 causes rotation of the filter 2 together with the rotary shaft 9. By centrifugal forces caused by rotation of the filter 2, the air in the housing body 14 can be blown outward radially.

The leg 16 is composed of four rod members extended downward from the lower wall 14b of the housing body 14. The drive section 4 is located in the center of those four rod members. According to the constitution that the housing body 14 is supported by the leg 16, the air outside of the housing body 14 can be drawn into through the minute holes 8 of the lower wall 14b of the housing body 14 without being interrupted by the leg 16.

As shown in Fig. 3B, within the housing body 14 and between the filter 2 and the hemispherical member 14c of the housing body 14, there is provided a diffuser guide 19 for adjusting a flow of the air in the housing body 14. The diffuser guide 19 comprises a plurality of annular plate members disposed equidistantly in the vertical direction and differing in width in the horizontal direction. Each of the annular plate members extends horizontally from the filter 2 toward the hemispherical portion 14c of the housing body 14 and is inclined at an outside edge portion to be substantially perpendicular to the hemispherical portion 14c. The diffuser guide 19 having such a constitution can guide uniformly the air purified through the filter 2 to the entire inside surface of the diffuser member 7.

As shown in Fig. 4A, the respective annular plate members composing the diffuser guide 19, are supported by four support plates 21 bridged from the upper wall 14a to the lower wall 14b of the housing body 14. The four support plates 21 are arranged with inclined by predetermined angle from the direction perpendicular to the periphery of the filter 2, when viewed from above. In other words, the four support plates 21 arranged with inclined to be along the blowing-out directions of the air from the filter 2.

Under the above-described constitution, in the air cleaner 15 relating to the present embodiment, rotation of the filter 2 by the drive section 4 causes rotation of the air in the filter 2 together with rotation of the filter 2, thereby generating centrifugal forces by which radially outside air flow is generated in the direction from the inside to the outside of the filter 2. Thus, the air surrounding the air cleaner 15 is sucked in through the upper wall 14a and the lower wall 14b of the housing body 14. This air passes through the filter 2 and is purified. The purified air is guided by the diffuser guide 19 and reaches the hemispherical portion 14c of the housing body 14. At this time, though the purified air flow blown out of the rotating filter 2 has swirling component, the amount of the air flow is adjusted by the diffuser guide 19 and guided uniformly to the hemispherical portion 14c of the housing body 14. And, the purified air reached the hemispherical portion 14c is diffused and blown out of the air cleaner 15 through the hemispherical portion 14c. Thus, the air cleaner 15 relating to the present embodiment can purify the surrounding air.

Here, in the air cleaner 15 relating to the present embodiment, an inlet opening of the air is formed by the upper wall 14a and the lower wall 14b of the housing body 14, and an outlet opening for blowing the purified air is formed by the hemispherical member 14c. The inlet opening and the outlet opening are adjacent to each other. In other words, the first design requirement is satisfied.

Further, in the air cleaner 15 relating to the present embodiment, the purified air is blown out by the hemispherical member 14c in the direction perpendicular to the curved surface of the hemispherical member 14c with uniformed flow flux, so the second requirement also is satisfied.

The air cleaner 15 relating to the present embodiment satisfies the first design requirement and the second design requirement, and attains the similar advantageous effects as those of the air cleaner 1 relating to the first embodiment.

In particular, the air cleaner 15 relating to the present embodiment is so configured that the filter 2 plays a role of a fan mechanism, so it is possible to reduce a volume occupied by a fan mechanism in the housing body 14 in comparison with the air cleaner 1 relating to the first embodiment. Moreover, an ordinary air cleaner is so designed that air speed passing through a filter is small in order to reduce pressure loss. According to the air cleaner 15 of the present embodiment, it is possible to maintain small speed of air flow by guiding the purified air blowing out through the side face of the filter 2 to the hemispherical portion 14c of the housing body 14 directly. Accordingly, it is possible to satisfy the first and the second design requirements superbly.

Incidentally, in the air cleaner 15 relating to the present embodiment, a constitution of the support plates 21 supporting the diffuser guide 19 in the housing body 14, is not limited to that explained above. For example, in stead of the supporting plates 21, feather-shaped supporting plates 22, as shown in Fig. 4B, may be adopted. Fig. 4B shows a modified example of the supporting plates 21 for supporting the diffuser guide 19 within the housing body 14 in the air cleaner 15 relating to the second embodiment.

The supporting plates 22 shown in Fig. 4B are arranged, in a similar manner to the support plates 21, to be along the blowing-out directions of the air from the filter 2, and to be inclined by a predetermined angle from the direction perpendicular to the outer peripheral surface of the filter 2, when viewed from above. The outside portion 22a of the supporting plate 22 when viewed from the rotary shaft 9, is bent toward radially outside and becomes thin toward outside tip end. By having such a configuration, the supporting plate 22 can guide the air blowing out through the rotating filter 2 and remove swirl components in the air.

The inner side portion 22b of the supporting plate 22 is rounded, when viewed from the rotary shaft 9. In the air cleaner 15 relating to the present embodiment, change in the number of rotation of the filter 2 causes increase or decrease in amount of the air flow blown out through the filter 2 and also change in blowing direction of the air. Even in a case where the blowing-out direction of the air has changed, the rounded-shaped inner side portion 22b of the supporting plate 22 can effectively prevent the air detachment at the inner side portion 22b impeding the air flow.

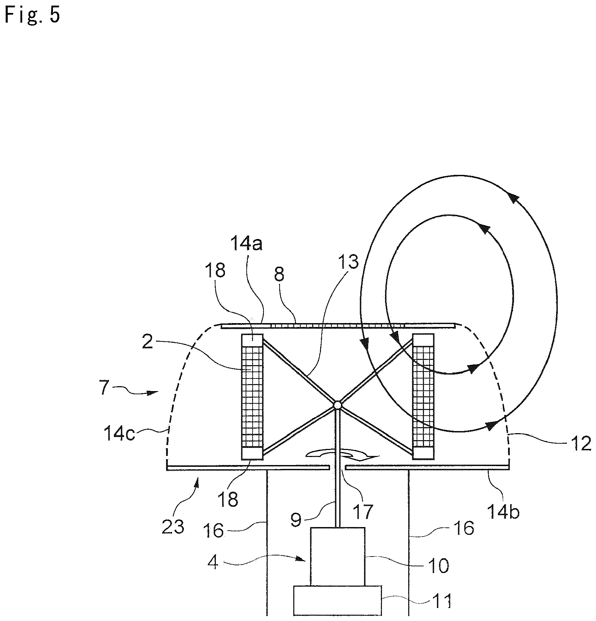

Meanwhile, in the air cleaner 15 relating to the present embodiment, the constitution of the housing body 14 is not limited to the aboves. For example, in stead of the housing body 14, a housing body 23, as shown in Fig. 5, may be adopted. Fig. 5 shows a modified example of the housing body 14 in the air cleaner 15 relating to the second embodiment.

The housing body 23 in Fig. 5 is the one having a circular upper wall 14a and a circular lower wall 14b that is coaxial with the upper wall 14a and larger in diameter than the upper wall 14a. More in detail, the housing body 23 is configured to be composed of two substantially hemispherical members connecting upper side and lower side which are parallel to each other in cross section along the center axis of the housing body 23, the lower side being longer than the upper side. Further, the lower wall 14b is formed with no micro openings, so outside air of the housing body 23 can be sucked in only through a number of micro openings 8 formed through the upper wall 14a. Meanwhile, diffuser guide 19 unillustrated is provided in the housing body 6.

By adopting the housing body 23 having such constitution as described above, the purified air blown out through the upper portions of the hemispherical portions 14c can be circulated, depicting small circles as shown in Fig. 5, and the purified air blown out through the lower portions of the hemispherical portions 14c can be circulated depicting large circles, so it is possible to purify the air surrounding the air cleaner 15, particularly, the upper air effectively.

### (Third embodiment)

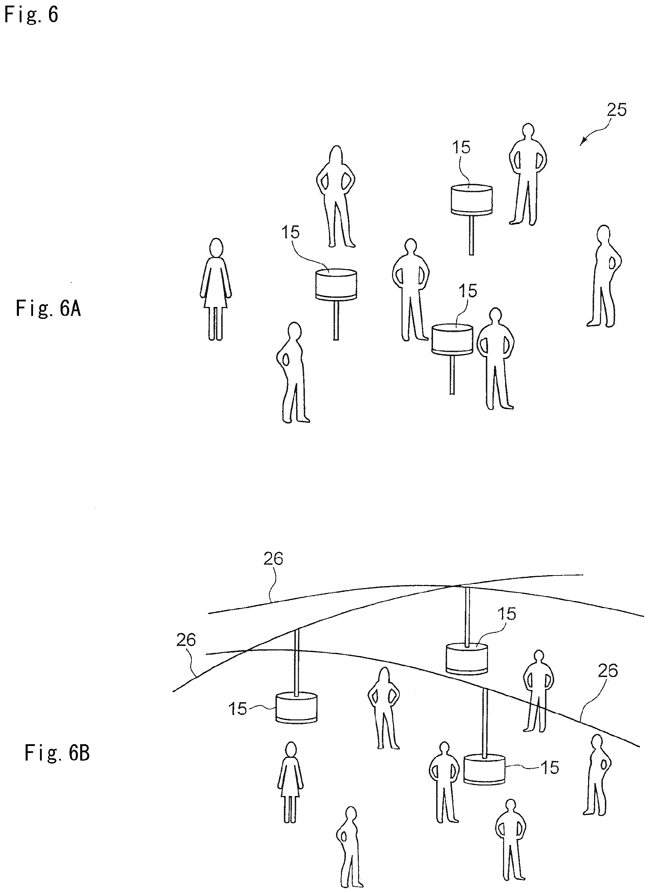

Fig. 6A is a view showing an air cleaning system relating to a third embodiment of the present invention.

As shown in Fig. 6A, an air cleaning system 25 has a plurality of the air cleaners 15 relating to the second embodiment disposed on the floor, for respectively being able to purify the surrounding airs locally. A plurality of the air cleaners 15 is dispersedly arranged in between a plurality of people in a space where people gather. By such arrangement, air between person and person can be locally purified, so the person and the person are separated by the air purified by the air cleaner 15 disposed therebetween. Thus, generation of air flow circulating in the entire room can be prevented, so virus infection to persons located downstream is hard to occur. In other words, the conventional air cleaner that purifies wide range of air in a room is apt to conduct cleaning or purifying air located at the ceiling or corners of room where no person is present. On the contrary, the air cleaning system 25 relating to the present embodiment can conduct air cleaning of only the portion or area where people gather. The air cleaner 15 can secure enlarged blowing-out path by which cross-section area of the flow path is increased and volume of the purified air in the space where people gather is enlarged, so that virus infection from person to person can be effectively reduced.

Incidentally, in the air cleaning system 25 relating to the present embodiment, in stead of a plurality of the air cleaners 15, a plurality of the air cleaners 1 relating to the first embodiment, or a plurality of the air cleaners 30, 35 or 40 relating to the fourth to sixth embodiments described later, can be adopted.

Further, in the air cleaning system 25 relating to the present embodiment, the arrangement of the plurality of the air cleaners 15 is not limited to the one disposed on the floor.

Fig. 6B shows a modified example of the air cleaning system 25 relating to the third embodiment. As shown in Fig. 6B, a holding member 26 for hanging and holding the air cleaner 15 may be provided above a space where people gather, and a plurality of the air cleaners 15 may be hung by the holding member 26. Such constitution can attain the similar effect to that attained by the above-described constitution that the air cleaners are disposed on the floor.

### (Fourth embodiment)

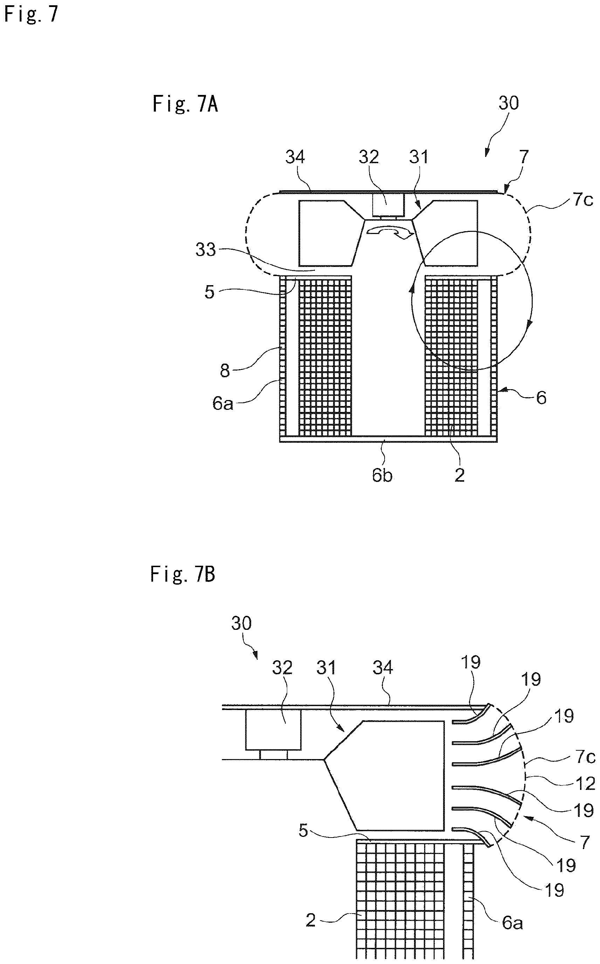

Fig. 7A is a sectional view showing a constitution of an air cleaner relating to a fourth embodiment of the present invention.

Regarding the air cleaner 30 shown in Fig. 7A, the same references are used for the same constitution as those of the first and second embodiments and the explanations thereto are omitted, and differing constitution only will be explained.

As shown in Fig. 7A, the air cleaner 30 comprises a filter 2, a centrifugal fan 31 disposed above the filter 2, a drive section 32 for driving the centrifugal fan 31, a partition member 5, a housing body 6 for receiving them and a diffusion member 7.

The air cleaner 1 relating to the first embodiment adopts an axial flow type fan 3. In comparison therewith, the air cleaner 30 relating to the present embodiment adopts a centrifugal type centrifugal fan 31 that is advantageous for a large amount of air flow and reduced noises.

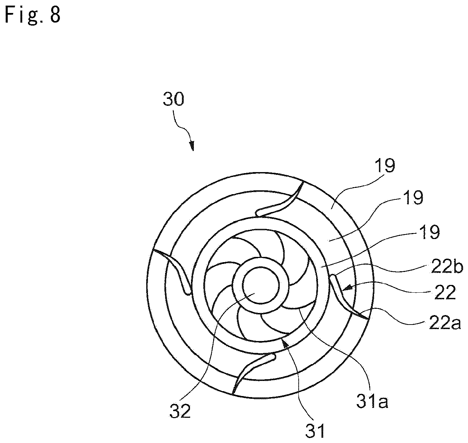

The centrifugal fan 31 is composed of an annular rotation body equipped with a plurality of blades 31a disposed along rotation directions (shown in Fig. 8). Rotation of the annular rotation body causes rotation of air locating between the blades 31 and generates centrifugal forces of the air, thus inducing air flow from inside to outside the rotation body. Meanwhile, a clearance 33 is provided between the centrifugal fans 31 and the partition member 5 to prevent the rotating centrifugal fans 31 from contacting the partition member 5.

The drive section 32 comprises a motor for driving the centrifugal fan 31 and a power source for supplying an electric power to the motor, in the same manner as the drive section 4 in the first embodiment. The driving section 32 is disposed above the centrifugal fan 31. The rotation center of the centrifugal fan 31, the filter 2 and the side wall 6a are coaxial.

The diffusion member 7 is similar to the housing body 14 in the second embodiment and hollowed, flat and spherically shaped. The upper face of the diffusion member 7 is closed by the upper wall 34 through which no air can pass, differing from the second embodiment, and the drive section 32 is fixed to the inside face of the upper wall 34. Meanwhile, the under face of the diffusion member 7 is opened and attached to the upper end of the housing body 6.

Within the diffusion member 7 as shown in Fig. 7B, the diffuser guides 19 which are similar to those in the second embodiment, are provided between the centrifugal fan 31 and the hemispherical portions 7c of the diffusion members 7. As shown in Fig. 8, the diffuser guides 19 are supported by the feather- shaped support plates 22 shown in Fig. 4B explained as the modification of the second embodiment.

Under the constitution as above described, in the air cleaner 30 relating to the present embodiment, the centrifugal fan 31 is rotated by the drive section 32, so that the air surrounding the air cleaner 30 is drawn in through the side wall 6a of the housing body 6. This air is passed through the filter 2 and purified. The purified air is guided upwardly by the centrifugal fan 31 and blown out from inside to outside the centrifugal fan 31, that is, radially outwardly. The thus purified air is guided by the diffuser guides 19 and reaches the hemispherical portions 7c of the diffusion members 7.

Meanwhile, the flow of the purified air blown out of the rotating centrifugal fan 31 has swirl components. The swirl components are removed effectively by the support plates 22, and the air flow is uniformly reduced in speed and guided to the hemispherical portions 7c of the diffusion member 7 by the diffuser guides 19. The purified air flow that has reached the hemispherical portions 7c, is diffused through the hemispherical portions 7c and blown out to the exterior of the air cleaner 30.

Thus, the air cleaner 30 relating to the present embodiment can purify the surrounding air.

Here, in the air cleaner 30 relating to the present embodiment, the side wall 6a of the housing body 6 constitutes an inlet opening of the air, and the hemispherical portion 7c of the diffusion member 7 constitutes an outlet opening for blowing out the purified air. The inlet opening and the outlet opening are adjacent to each other, so the first design requirement is satisfied.

Further, in the air cleaner 30 relating to the present embodiment, the purified air is blown out by the hemispherical portion 7c in the direction perpendicular to the curved surface of the hemispherical portion 7c with uniformed flow flux, so the second requirement also is satisfied.

The air cleaner 30 relating to the present embodiment satisfies the first design requirement and the second design requirement, and can attain the similar advantageous effects as those of the air cleaner 1 relating to the first embodiment.

In particular, the air cleaner 30 relating to the present embodiment adopts the centrifugal fan for the air blowing mechanism. Therefore, in comparison with the air cleaner 1 relating to the first embodiment provided with the axis-flow type fan 3, the air cleaner 30 relating to the present embodiment can attain a large air flow, improve a cleaning ability and realize reduction of noises.

### (Fifth embodiment)

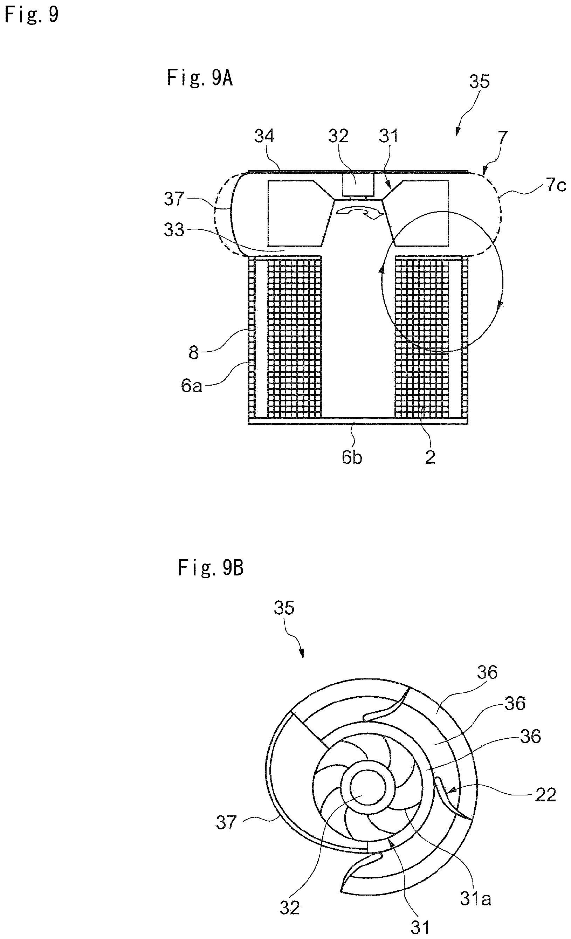

Fig. 9A is a sectional view showing a constitution of an air cleaner relating to a fifth embodiment of the present invention.

Regarding the air cleaner 35 shown in Fig. 9A, the same references are used for the same constitution as those of the fourth embodiment and the explanations thereto are omitted, and differing constitution only will be explained.

As shown in Fig. 9B, the air cleaner 35 comprises diffuser guides 36 and shells (guide plates) 37, in stead of the diffuser guide 19 in the fourth embodiment.

The diffuser guides 36 are so configured that the respective annular members composing the diffuser guide 19, are cut away to be C-shaped, at the same portion seen from above, and in detail, from the neighborhood of the rear side of an arbitrary support plate 22 to a portion between the second and third support plates 22, and more in detail, by 135 degrees, seen from the center.

The shell 37 is a U-shaped plate member loosely curved in cross section like a shell. The shells 37 are so disposed over the cut-away portions of the respective annular plate members composing the diffuser guides 36 to cover the cut-away portions. In more detail, an end of the shell 37, as shown in Fig. 9B, is disposed in the vicinity of an inside end of the arbitrary support plate 22, and the other end of the shell 37 is disposed in the vicinity of the outer side end of the annular plate member.

Under such constitution, the air cleaner 35 relating to the present embodiment attains the similar advantageous effects as those of the air cleaner 30 relating to the fourth embodiment.

In particular, in the air cleaner 35 relating to the present embodiment, the purified air advancing toward the shell 37 in the purified air blown out by the centrifugal fan 31, is guided by the shells 37 in the direction of rotation of the centrifugal fan 31 and is led to the cut-away ends of the respective annular plate members. Thus, the purified air is guided by the respective annular plate members, diffused through the hemispherical portions 7c of the diffusion member 7, and blown out to the exterior of the air cleaner 35. Thus, the air cleaner 35 can lead the purified air advancing toward the shells 37, to the diffuser guides 19 with no loss, and to the outside of the air cleaner 35. And, air flow can be prevented from being generated on the rear side of the shells 37 when viewed from the rotation center of the centrifugal fan 31. Accordingly, when the air cleaner 35 is used near a wall or a back of a chair, for example, it is possible to prevent the purified air from being impinged on the wall or the back of the chair by disposing the air cleaner 35 so that the back sides of the shells 37 may be located near the wall or the back of the chair, thereby the air flow in unintentional direction can be prevented. As a result, it is possible to effectively prevent diffusion of viruses.

### (Sixth embodiment)

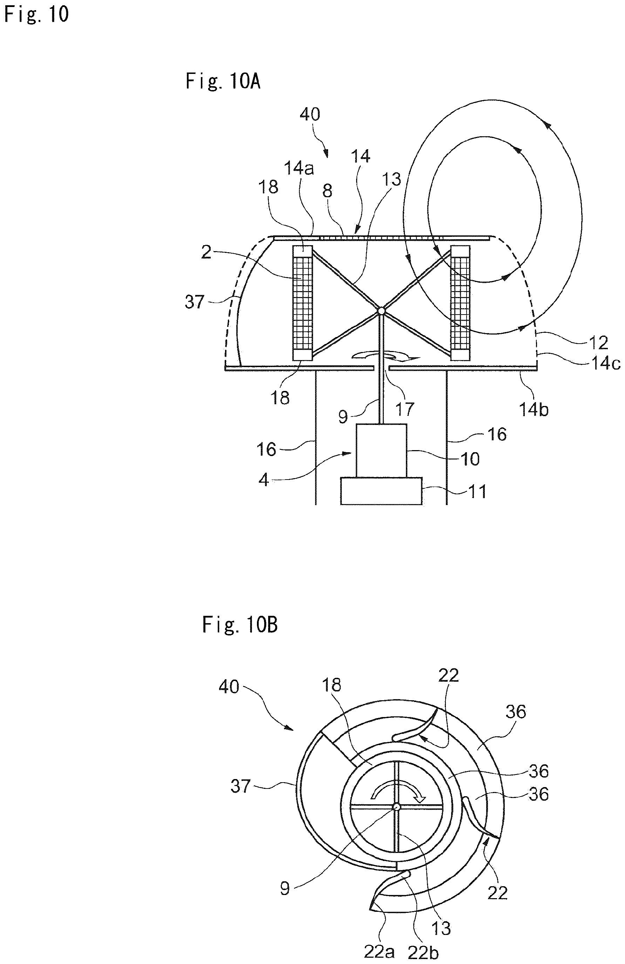

Fig. 10A is a sectional view showing a constitution of an air cleaner relating to a sixth embodiment of the present invention.

Regarding the air cleaner 40 shown in Fig. 10A, the same references are used for the same constitution as those of the second and fifth embodiments and the explanations thereto are omitted, and differing constitution only will be explained.

The air cleaner 40 shown in Fig. 10A, is the one in which the diffuser guides 36, the shells 37 and support plates 22 described in the fifth embodiment are applied to an air cleaner relating to a modification of the second embodiment, shown in Fig. 5.

Under such constitution, the air cleaner 40 relating to the present embodiment attains the similar advantageous effects as those of the air cleaner 15 relating to the second embodiment.

Further, in a similar manner to the air cleaner 35 of the fifth embodiment, when the air cleaner 40 relating to the present embodiment is disposed near, for example, a wall or a back of a chair, flow of the purified air in the unintentional direction due to collision of the purified air flow with the wall or the back of the chair can be prevented from being caused, as a result diffusion of viruses being effectively prevented.

As described above, according to the respective embodiments, an air cleaner, an air cleaning method and an air cleaning system, which are suitable for preventing infection of virus from person to person.

### EXPLANATION OF REFERENCES

- 1, 15, 30, 35, 40: air cleaner
- 2: filter
- 3: fan
- 4, 32: drive portion
- 6, 14, 23: housing body
- 7: diffusion member
- 8: micro holes
- 9: rotary shaft
- 10: motor
- 11: power source
- 12: hole
- 13: supporting bar
- 16: leg
- 18: plate member
- 19, 36: diffuser guide
- 21, 22: support plate
- 25: air cleaning system
- 31: centrifugal fan
- 31a: blade of centrifugal guide
- 37: shell

## Claims

1. An air cleaner comprises:
a housing body provided with an inlet opening for sucking an external air;
a filter disposed in said housing body and for purifying the air;
a blowing device received in the housing body to blow out the air;
a drive portion for rotation-driving said blowing device; and
a diffusion member provided to the housing body as an outlet opening for blowing out the purified air purified through said filter to outside, wherein
said inlet opening and said outlet opening are adjacent to each other; and
the air purified through said filter is diffused by said diffusion member and blown out.

2. The air cleaner according to claim 1, wherein the diffusion member is honeycomb formed with hexagonal through-holes each extended to be converged toward the inner side surface from the outer side surface of the diffusion member.

3. The air cleaner according to claim 1 or claim 2, wherein
said housing body is cylindrical;
said inlet opening is provided on the side surface of said cylindrical housing body;
said filter is coaxial with said housing body and cylindrical smaller in diameter than said housing body;
said blowing device includes a fan disposed in said filter; and
said diffusion member is disposed at an end of the cylindrical housing body and is hollowed hemispherical.

4. The air cleaner according to claim 1 or claim 2,
wherein
said housing body is cylindrical;
said inlet opening is provided on the side surface of said cylindrical housing body;
said filter is coaxial with said housing body and cylindrical smaller in diameter than said housing body;
said blowing device includes a centrifugal fan disposed above said filter;
said diffusion member is disposed at an upper end of the cylindrical housing body and is hollowed, flattened spherical shaped for receiving the centrifugal fan; and
the upper face of said diffusion member is closed so as not to pass the air.

5. The air cleaner according to claim 1 or
claim 2, wherein
said housing body is hollowed, flat and spherical;
said inlet opening is provided at least on an upper surface of the upper surface and a lower surface of the side surface of said housing body;
said filter is coaxial with said housing body and cylindrical smaller in diameter than said housing body; and
said blowing device includes said filter and blows out the air to the exterior radially outwardly by rotating said filter by said drive portion.

6. The air cleaner according to claim 5,
wherein
said housing body is shaped to have a cross section along the center axis of said housing body composed of parallel upper and lower sides which are same in length and connected with two hemispheres; and
said inlet opening is provided on the upper and lower surfaces of said housing body.

7. The air cleaner according to claim 5, wherein said housing body is shaped to have a cross section along the center axis of said housing body composed of parallel upper and lower sides and connected with two substantially hemispheres, said lower side being larger in length than said upper side; and
said inlet opening is provided on only the upper surface of said housing body.

8. The air cleaner according to any one of claim 4 to claim 7, wherein
diffuser guide for leading the air purified by said filter to over the entire inner side surfaces of said diffusion member is provided between said filter and said diffusion member or between said centrifugal fan and said diffusion member.

9. The air cleaner according to claim 8,
wherein said diffuser guide includes a plurality of annular plate members arranged spaced apart in the vertical direction, the plurality of annular plate members extending horizontally and having outer edges inclined to be perpendicular to said diffusion member.

10. The air cleaner according to claim 9, wherein
the plurality of said annular plate members are cut away to be C-shaped at the same portions when viewed from above,
the cut-away portions of the plurality of said annular plate members are provided with guide members for guiding the purified air blown out to the exterior radially outwardly, into the direction of rotation of said blower device.

11. The air cleaner according to any one of claim 8 to claim 10, wherein
said diffuser guide is supported to the plate member for removing swirl component of the purified air.

12. An air cleaning method **characterized in** arranging between person and person the air cleaner according to any one of claim 1 to claim 11.

13. An air cleaning system **characterized in** distributed arrangement of a plurality of the air cleaners according to any one of claim 1 to claim 11.

14. An air cleaning system being **characterized in** distributed arrangement of a plurality of the air cleaners each purifying surrounding air locally.

## Amended claims

### Amended claims under Art. 19.1 PCT

1. (amended) An air cleaner comprises:
a housing body provided with an inlet opening for sucking an external air;
a filter disposed in said housing body and for purifying the air;
a blowing device received in the housing body to blow out the air;
a drive portion for rotation-driving said blowing device; and
a diffusion member provided to the housing body as an outlet opening for blowing out the purified air purified through said filter to outside, wherein
said housing body is cylindrical and provided with said inlet opening on the side surface of said housing body, said housing body receiving therein said filter that is coaxial with said housing body and cylindrical smaller in diameter than said housing body, said blower device being disposed on an upper end of said filter, and said diffusion member being disposed on an end of the housing body, thereby said inlet opening and said outlet opening being arranged adjacent to each other; and
the air drawn in from through said inlet opening, after passing through inside said filter, purified through said filter, is led to said blowing device, sent to said diffusion member by said blowing device, and diffused by said diffusion member and blown out to be circulated into said inlet opening.

2. (amended) An air cleaner comprises:
a housing body provided with an inlet opening for sucking an external air;
a filter disposed in said housing body and for purifying the air;
a blowing device received in the housing body to blow out the air;
a drive portion for rotation-driving said blowing device; and
a diffusion member provided to the housing body as an outlet opening for blowing out the purified air purified through said filter to outside, wherein
said housing body is hollowed, flat and spherical and provided with said inlet opening at least on an upper surface of the upper surface and a lower surface of said housing body;
said filter is coaxial with said housing body and cylindrical smaller in diameter than said housing body;
said diffusion member is disposed on the side surface of the housing body, thereby said inlet opening and said outlet opening are arranged adjacent to each other; and
said blower device includes said filter, and
the air drawn in from through said inlet opening, and led to inside said filter, after passing through said filter by rotating said filter by said drive portion, purified through said filter, and sent radially outside, and diffused by said diffusion member and thereafter blown out to be circulated into said inlet opening.

3. (amended) The air cleaner according to claim 1, wherein
said blower device includes a fan; and
said diffusion member is hollowed hemispherical.

4. (amended) The air cleaner according to claim 1, wherein
said blowing device includes a centrifugal fan; and
said diffusion member is hollowed, flattened and spherical shaped for receiving the centrifugal fan; and
the upper surface of said diffusion member is closed so as not to pass the air.

5. (amended) The air cleaner according to claim 2, wherein
said housing body is shaped to have a cross section along the center axis of said housing body composed of parallel upper and lower sides which are same in length and connected with two hemispheres; and
said inlet opening is provided on the upper and lower surfaces of said housing body.

6. (amended) The air cleaner according to claim 2, wherein
said housing body is shaped to have a cross section along the center axis of said housing body composed of parallel upper and lower sides and connected with two substantially hemispheres, said lower side being larger in length than said upper side; and
said inlet opening is provided on only the upper surface of said housing body.

7. (amended) The air cleaner according to any one of claim 4 to claim 6, wherein
diffuser guide for leading the air purified by said filter to over the entire inner side surface of said diffusion member, is provided between said filter and said diffusion member or between said centrifugal fan and said diffusion member.

8. (amended) The air cleaner according to claim 7, wherein
said diffuser guide includes a plurality of annular plate members arranged spaced apart in the vertical direction, the plurality of annular plate members extending in the horizontal direction and having outer edges inclined to be perpendicular to said diffusion member.

9. (amended) The air cleaner according to claim 8, wherein
the plurality of said annular plate members is cut away to be C-shaped at the same portions when viewed from above,
the cut-away portions of the plurality of said annular plate members are provided with guide members for guiding the purified air blown out to the exterior radially outwardly, into the direction of rotation of said blowing device.

10. (amended) The air cleaner according to any one of claim 7 to claim 9, wherein
said diffuser guide is supported to the plate member for removing swirl component of the purified air.

11. (amended) The air cleaner according to any one of claims 1 to 10, wherein the diffusion member is honeycomb formed with hexagonal through-holes each extended to be converged toward the inner side surface from the outer side surface of the diffusion member.

12. (amended) An air cleaning method **characterized in** arranging between person and person the air cleaner according to any one of claim 1 to claim 11.

13. (amended) An air cleaning method **characterized in** distributed arrangement of a plurality of the air cleaners each according to any one of claim 1 to claim 11.

14. (deleted)

Statement under Art. 19.1 PCT
The applicant, who received the International Search Report relating to the above identified International Application transmitted on 22. 11. 2021, hereby files amendment under Article 19(1) as in the attached sheet.

The applicant hereby would like to amend the claims 1 to 13, and cancel the claim 14.

The claim 1 is so amended that, to the originally filed claim(hereinafter called as "original claim") 1, are added common portions in the original claims 3, 4 and limitations based on paragraphs [0023] to [0025] and [0049] to [0051].

The claim 2 is a new independent claim in which, to the original claim 1, the original claim 5 and limitations based on paragraphs [0032] to [0034] and [0038] to [0040], are added.

The claim 3 is one in which the common portions in the original claims 3,4 added to the claim 1 are deleted from the original claim 3.

The claim 4 is one in which the common portions in the original claims 3,4 added to the claim 1 are deleted from the original claim 4.

The claims 5-10 are renumbered ones of the original claims 6-11.

The claim 11 is the same as the original claim 2.

The claim 12 and 13 are the same as the original claims 12 and 13.
